# EUROPEAN PATENT SPECIFICATION

(11) **EP 0 367 335 B1**
(45) Date of publication and mention of the grant of the patent: **15.06.1994**
(21) Application number: 89202679.0
(22) Date of filing: 24.10.1989
(51) Int. Cl.: A61K 7/50, A61K 7/48, C11D 3/37

(54) **Toilet bar composition containing cationic guar gum**
Kationische Guargummi enthaltende Toilettenseifenzusammensetzung
Composition de barre de toilette contenant une gomme de guar cationique

(30) Priority: 02.11.1988 US 266039
(43) Date of publication of application: 09.05.1990
(73) Proprietor: THE PROCTER & GAMBLE COMPANY, Cincinnati, Ohio 45202 (US)
(72) Inventor: Knochel, John Robert, Cincinnati Ohio 45242 (US); Vest, Pauld Edward, Cincinnati Ohio 45255 (US)
(74) Representative: Canonici, Jean-Jacques

(56) References cited:
- EP-A- 0 222 525
- EP-A- 0 227 321
- DE-A- 3 413 475
- MANUFACTURING CHEMIST. vol. 57, no. 11, November 1986, LONDON GB pages 79 - 83; Philip Alexander: "Organic Rheological Additives"

## Description

This invention pertains to a process for making personal cleansing toilet bar compositions, which comprise cationic guar gum.

| U.S. Patent Documents | | | |
|---|---|---|---|
| Pat. No. | Date | Inventor(s) | U.S. Class/Sub. |
| 3,761,418 | 9/1973 | Parran, Jr. | 252/106 |
| 4,234,464 | 11/1980 | Morshauser | 252/544 |
| 4,061,602 | 12/1977 | Oberstar et al. | 252/547 |
| 4,472,297 | 9/1984 | Bolich et al. | 252/531 |
| 4,491,539 | 1/1985 | Hoskins et al. | 252/541 |
| 4,540,507 | 9/1985 | Grollier | 252/174.23 |
| 4,673,525 | 6/1987 | Small et al. | 252/132 |
| 4,704,224 | 11/1987 | Saud | 252/132 |

### Other Documents

Jap. J57105 6/30/82 Pola
Cosmetics & Toiletries, Vol. 99, June 1984, pp. 83-87, Allured Publishing Co., "Cationic Guar Gum," by Freeland et al.

Toilet bars based on soap (alkali metal salts of fatty acids) are commonly used for cleansing the human body. A wide variety of additives have been suggested for inclusion in toilet bars. Some enhance the physical properties of the bar (hardness, wear rate, resistance to water). Others enhance the in-use properties of the toilet bar (lather characteristics such as volume and texture), and some impact on the impression the bar has on the skin both during washing (bar feel) and afterwards.

It has been discovered that the addition of certain polymeric materials to toilet bars can have a beneficial skin mildness effect for the user without deleteriously affecting other bar properties. On the other hand, some polymeric materials are very difficult to incorporate into bars and result in a gritty in-use skin feel.

Prior art cationic polymers include cationic guar gums such as hydroxyproxyltrimethylammonium guar gum such as that available commercially under the trademarks Jaguar C-17 (RTM) and Jaguar C-15 (RTM) as marketed by Hi-Tek Polymers of Louisville, Ky.

The problem with using prior art slow hydrating cationic guar gums in soap bars is that they present costly processing problems and often result in gritty feeling bars. The use of prior art slow hydrating cationic guar gums to dried soap flakes, i.e., at the milling step, results in inconsistent and unacceptable bar feel. EP-A-0 227 321, Medcalf, Jr., Visscher, Knochel and Dahlgren, discloses a soap bar comprising hydrated cationic guar gum. Medcalf, Jr., et al., addressed this serious gritty feeling problem associated with making soap bars using cationic guar gum. Medcalf, Jr., et al. teaches that the gritty feeling problem is solved by insuring that the cationic guar gum is hydrated and well incorporated into the soap bar matrix. However, the cationic guar gums specifically disclosed in Medcalf, Jr., et al., if added to dried soap flakes, do not hydrate rapidly enough to avoid the gritty feeling problem.

EP-A-0 222 525 A3, Dawson and Williams, discloses a beta-phase soap bar comprising a water soluble polymer.
According to the present invention there is provided a process for preparing a toilet bar composition comprising the steps of:
(a) forming a mixture of cationic guar gum and alkali metal soap mix noodles wherein said guar gum is present at a level of from 0.2% to 5% by weight of said composition; said mixture containing from 8% to 12% by weight thereof of moisture;
(b) hydrating said cationic guar gum by milling said mixture into a uniform mixture;
(c) plodding said uniform mixture;
(d) extruding said milled and plodded mixture; and
(e) stamping said extruded mixture into said toilet bars,
and wherein said cationic guar gum is a fast hydrating cationic guar gum which at a level of 2.6% in 9.5% alkaline (pH 9.4) salt (NaCl) water solution at 25°C has a viscosity of at least 800 mPa.s in 10 minutes and at least 1,000 mPa.s in 30 minutes under the same conditions.

The soap component of the compositions made herein preferably comprises from 50% to 90% by weight alkali metal soap (anhydrous basis) and is an alkali metal (e.g., sodium or potassium) soap or mixture of soaps of fatty acids containing from 8 to 24, preferably from 10 to 20 carbon atoms. The fatty acids used in making the soaps can be obtained from natural sources such as, for instance, plant or animal-derived glycerides (e.g., palm oil, coconut oil, babassu oil, soybean oil, castor oil, whale oil, fish oil, tallow, grease, etc., and mixtures thereof. The fatty acids can also be synthetically prepared (e.g., by oxidation of petroleum stocks by the Fischer-Tropsch process).

Alkali metal soaps can be made by direct saponification of the fats and oils or by the neutralization of the free fatty acids which are prepared in a separate manufacturing process. Particularly useful are the sodium and potassium salts of the mixtures of fatty acids derived from coconut oil and tallow, i.e., sodium and potassium tallow and coconut soaps.

The term "tallow" is used herein in connection with fatty acid mixtures which typically have an approximate carbon chain length distribution of 2.5% C₁₄, 29% C₁₆, 23% C₁₈, 2% palmitoleic, 41.5% oleic and 3% linoleic. (The first three fatty acids listed are saturated.) Other mixtures with similar distribution, such as the fatty acids derived from various animal tallows and lard, are also included within the term tallow. The tallow can also be hardened (i.e., hydrogenated) to convert part or all of the unsaturated fatty acid moieties to saturated fatty acid moieties.

When the terms "coconut oil" and "coconut fatty acid" (CNFA) are used herein, they refer to fatty acid mixtures which typically have an approximate carbon chain length distribution of about 8% C₇, 7% C₁₀, 48% C₁₂, 17% C₁₄, 9% C₁₆, 2% C₁₈, 7% oleic, and 2% linoleic. (The first six fatty acids listed are saturated.) Other sources having similar carbon chain length distribution such as palm kernel oil and babassu kernel oil are included with the terms coconut oil and coconut fatty acid.

In the compositions made herein, the soap component is preferably either sodium soap or a mixture of sodium and potassium soap wherein the mixture contains no more than 25% by weight potassium soap.

Also, it is preferable in such bars that the total soap component comprises (a) from 20% to 80% by weight of the soap component of a mixture containing soaps having from 8 to 14 carbon atoms and (b) from 20% to 80% by weight of the soap component of soaps having from 16 to 20 carbon atoms.

Soaps having such preferred chain length distribution characteristics can be realized by utilizing mixtures of tallow and coconut fatty acids in tallow/coconut (T/CN) weight ratios varying between 90:10 and 50:50. A mixture of soaps of tallow and coconut fatty acids in the tallow/coconut weight ratio of 80:20 is especially preferred. A preferred soap bar also comprises from at least 25% of tallow soap. Another preferred bar comprises a 50%/80% T/CN fatty acid soap mixture.

A preferred soap bar comprises about 50% soap as its primary or sole surfactant It also contains as an essential ingredient a hydrated, cationic guar gum provided by a "fast hydrating" cationic guar gum polymer. This polymer is uniformly distributed in the soap bar matrix without affecting the smooth feel of the dry or wet bar and improves the skin feel of the bar when compared to a comparably processed soap bar with a slow hydrating guar gum polymer. The mildness achieved approaches that of toilet bars based on mild synthetic surfactants as disclosed in U.S.-A-4,673,525, Small et al., issued June 16, 1987. Yet the preferred bar maintains the highly acceptable physical and in-use characteristics of a pure soap bar.

Synthetic detergents can also be present in compositions herein. Preferred types of synthetic detergents are of the anionic or nonionic type. Examples of anionic synthetic detergents are the salts of organic sulfuric reaction products such as
alkyl sulfates having the formula R₂₄OSO₃M;
alkyl sulfonates having the formula R₂₄SO₃M;
alkyl ether sulfates having the formula R₂₄(OC₂H₄)ₓOSO₃M;
alkyl mono glyceride sulfonates having the formula 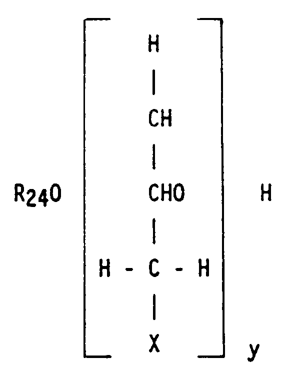
and alkyl benzene sulfonates having the formula 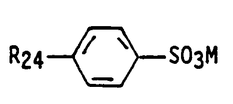
In the above formulae, R₂₄ is a straight or branched chain alkyl of from 8 to 24 carbon atoms; M is an alkali metal or ammonium ion; x is a number of from 1 to 10; y is a number of from 1 to 4; and X is selected from the group consisting of chlorine, hydroxyl, and -SO₃M, at least one X in each molecule being -SO₃M. Examples of nonionic synthetic detergents are ethoxylated fatty alcohols (e.g., the reaction product of one mole of coconut fatty alcohol with from about 3 to about 30 moles of ethylene oxide) and fatty acid amides such as coconut fatty acid monoethanolamide and stearic acid diethanolamide. Although it may be desirable in some instances to incorporate synthetic detergents the compositions made herein can be free of synthetic detergents. Preferred are the mild synthetic surfactants disclosed in U.S.-A-4,673,525, Small et al., issued June 16, 1987.

Insoluble alkaline earth metal soaps such as calcium stearate and magnesium stearate can also be incorporated into compositions at levels up to 30%. These materials are particularly useful in toilet bars in which synthetic detergents are present in that they tend to reduce the relatively high solubility which such bars normally have. These alkaline earth metal soaps are not included within the term "soap" as otherwise used in this specification. The term "soap" as used herein refers to the alkali metal soaps.

Powdered or dispersed fast hydrating cationic guar gum polymer is uniformly dispersed and incorporated into the soap bar formulation by mixing same with dried soap flakes, i.e., at the mixing/milling step.

The soap bar comprises 0.2% to 5%, of the fast hydrating cationic guar gum polymer. The molecular weight of the preferred cationic guar gum is from 50,000 to 1,000,000, preferably from 100,000 to 500,000, and more preferably from 250,000 to 400,000.

The essential component of the toilet bar made herein is the fast hydrating cationic guar gum as herein disclosed. The preferred guar gum has a viscosity profile of:
from 5 to 500 mPa.s (cps) in 5 minutes;
from 1,000 to 5,000 mPa.s (cps) in 30 minutes;
from 3,000 to 9,000 mPa.s (cps) in 60 minutes; and
from 5,000 to 14,000 mPa.s (cps) in 120 minutes.

Guar gum is a natural material derived from ground up endosperms of Cyamonsis tetragonolobus. Preferably, the guar gum used in the present invention is a free flowing powder having a particle size on a 150 mesh of from 0% to 10%, preferably from 3% to 5% maximum and through a 250 mesh of 50%, preferably 65%.

The fast hydrating cationic guar gum is incorporated into the soap composition (as described below) at a level of from 0.2% to 5%, preferably from 0.5% to 4%. The guar gum is surprisingly hydrated as it is introduced into the composition at the mixing/milling step of a conventional soap making process. Preferably, the composition comprises from 1% to 3% guar gum, more preferably from 1% to 2%.

**TABLE 1**

| Cationic Jaguar (RTM) Guar Gum Viscosities | | | | |
|---|---|---|---|---|
| Gum Samples | Viscosity mPa.s (cps) * | | | |
| | 5 minutes | 30 minutes | 60 minutes | 120 minutes |
| A | 100 | 2100 | 4200 | 5500 |
| B | 100 to 700 | 2150 to 5300 | 4400 to 9460 | 5600 to 12,000 |
| C | 15-20 | 75-170 | 215-420 | 560-1300 |
| D | 100 | 500 | 1500 | 2000 |
| E | 210 to 920 | 1000 to 2300 | 1800 to 3600 | 2600 to 4400 |
| F | 15 | 100 | 200 | 540 |

| | | | | |
|---|---|---|---|---|
| *The rate of hydration is defined in terms of the viscosity of a 2.6% guar gum polymer in a 9.5% NaCl alkaline (9.4 pH) water solution at 25°C. | | | | |

A preferred fast hydrating cationic guar gum has a viscosity of at least 2000 mPa.s (cps) in 30 minutes in the 2.6% cationic guar gum polymer/alkaline/salt water solution at 25°C.

The toilet bars generally contain from 8% to 20% by weight of water.

However, some toilet bars comprising a substantial amount of synthetic surfactant as its primary surfactant can contain as little as from 3.5% to 4.5% water.

The toilet bar compositions can contain optional components such as those conventionally found in toilet bars. Conventional antibacterial agents can be included in the present compositions at levels of from 0.5% to 4%. Typical antibacterial agents which are suitable for use herein are 3,4-di and 3,4',5-tribromosalicyla-anildes; 4,4'-dichloro-3-(trifluoromethyl)carbanilide; 3,4,4'-trichlorocarbanilide and mixtures of these materials. Conventional nonionic emollients can be included as additional skin conditioning agents in the compositions at levels up to 40%, preferably at levels of from 1% to 25%. Such materials include, for example, mineral oils, paraffin wax having a melting point of from about 37.7°C (100°F), fatty sorbitan esters (see U.S.-A-3,988,255, Seiden, lanolin and lanolin derivatives, esters such as isopropyl myristate and triglycerides such as coconut oil or hydrogenated tallow.

Free fatty acid such as coconut fatty acid can be added to the compositions herein to improve the volume and quality (creaminess) of the lather produced by the compositions herein.

Conventional perfumes, dyes and pigments can also be incorporated into compositions at levels up to 5%. Perfumes are preferably used at levels of from 0.5% to 3% and dyes and pigments are preferably used at levels of from 0.001% to 0.5%.

In the preferred process, powdered, fast hydrating cationic guar gum is added to noodles of the base soap mixture in an amalgamator. Any optional ingredients such as perfumes, dyes, etc., are also added to the amalgamator. The mixture is processed in the amalgamator and milled in the conventional manner under conventional conditions. It is then plodded and extruded into logs for cutting and stamping into toilet bars.

In the process of the present invention, the guar gum cationic polymer is hydrated with the moisture in the soap noodle mix in the soap mixing steps of the soap bar making process. Hydration of the polymer is fast. The fast hydrating polymer goes into the soap mixture readily and the polymer is distributed uniformly without significant numbers of nonhydrated polymer chunks. The uniform distribution of the polymer maintains highly acceptable soap bar feel in-use characteristics.

The soap bars preferably contain up to 20% of a synthetic surfactant. If a synthetic surfactant is included, a mild one is preferred. A mild synthetic surfactant is defined herein as one which does relatively little damage to the barrier function of the stratum corneum. The mild surfactant is preferably used at a level of 2-15%. The fatty acid soap and mild surfactant mixture preferably has a ratio of 2.5:1 to 37:1, preferably from 2.5:1 to 14:1, and most preferably from 6.5:1 to 14:1, soap:synthetic.

A preferred soap bar also contains from 2% to 17% moisturizer, preferably one selected from glycerin and free fatty acid or mixtures thereof. The more preferred bar contains at least 4% moisturizer.

Some preferred mild synthetic surfactants useful in this invention include alkyl glyceryl ether sulfonate (AGS), anionic acyl sarcosinates, methyl acyl taurates, N-acyl glutamates, alkyl glucosides, acyl isethionates, alkyl sulfosuccinate, alkyl phosphate esters, ethoxylated alkyl phosphate esters, alkyl ether sulfates, methyl glucose esters, protein condensates, mixtures of alkyl ethoxylated sulfates and alkyl amine oxides, betaines, sultaines, and mixtures thereof. Included in the surfactants are the alkyl ether sulfates with 1 to 12 ethoxy groups, especially ammonium and sodium lauryl ether sulfates. Alkyl chain lengths for these surfactants are C₈-C₂₂, preferably C₁₀-C₁₈. The most preferred mild surfactant is sodium coconut alkyl glyceryl ether sulfonate.

Hydration of the cationic guar gum polymer can be judged during the soap making process by examination of the soap mix after the polymer has been added and mixed for at least 10 minutes. It is preferred that no lumps of polymer be visible to the eye during this examination. Additionally, the feel of the resultant test soap mix must not be gritty or grainy upon evaluation in water. On a scale of 0 to 10, the bar should be 7 or better.

The following examples are presented by way of illustration only.

### EXAMPLE I

In general, procedures common to conventional toilet soap bar making are employed.

### Continuous Crutching Step

About 120.1 parts of a mix containing 29.4% moisture, 54.5% 50/50 tallow/coconut soap (T/CN), 7.3% coconut (CN) alkyl glycerol sulfonate (AGS), 3.3% glycerin, 0.7% NaCl, and approximately 1.5% miscellaneous are mixed in line. The mix temperature is approximately 150-200°F (65-94°C).

### Vacuum Drying and Plodding Steps

The mix is vacuum dried at about 6.7 kPa (50 mm Hg) absolute pressure to reduce its moisture to approximately 11%. The resultant dried soap is plodded into noodles.

### Mixing/Milling Steps

### Polymer Addition Step

The plodded noodles are conveyed to a continuous mixer (CM) where approximately 1.0 parts each of guar gum sample B (Table 1) and Merquat 550 (RTM) polymers introduced, mixed, and plodded with the soap noodles. Uniform distribution during this addition and mixing step and the absence of any liquid flows, especially glycerin, are important for acceptable bar feel performance. The polymer/soap noodles (generic noodles) are conveyed to milling.

### Generic Milling Step

Two four-roll soap mills (feed, stationary, middle, top rolls) are used in this step. This is a split milling (two set of mills are used in parallel) process to obtain an homogeneous mix. Efficient milling is needed in this intimate mixing step.

### Dry Mixing Step

The generic noodles are conveyed to a second process system continuous mixer (CM) for the addition and mixing of 1.5 parts of trichlorocarbanilide (TCC) and 0.24-0.55 parts of TiO₂. This mix is plodded and conveyed to the third process CM.

### Wet Mixing Step

1.2 parts of perfume, 1.1 parts of NaCl/diethylene triamine acetate (DTPA) solution, and 0.0 to 0.50 parts of color solution are added and mixed in this web mixing (CM) step. This finished soap formula is then plodded into soap noodles and conveyed via a transport plodder to a final milling step.

The mixture is milled using a four-roll mill, plodded, and then stamped into toilet bars of any convenient size and shape. The resulting bars demonstrate enhanced physical properties particularly good, bar feel properties and the skin mildness mentioned above.

### EXAMPLES II-VII

Toilet bars are prepared using the process of Example I, except that the cationic guar gums as set out in Table 2 are used. Their viscosity profiles are set out in Table 1.

**TABLE 2**

| Example | Gum Sample | Bar Feel |
|---|---|---|
| II | A | Excellent |
| III | B | Excellent |
| IV | C | Very Poor |
| V | F | Poor |
| VI | E | Good |
| VII | D | Poor |

Note that the bars of Examples II, III and VI according to the invention containing the fast hydrating cationic guar gums have much better bar feels than the slow hydrating guar gums used in Control bars IV, V an VII.

Compared to toilet bars which are prepared with slow hydrating cationic guar gum added dry to dried soap noodles or flakes at the mixing/milling steps of a conventional bar soap making process, the toilet bars consistently exhibit superior bar feel due to the fast hydrating cationic guar gum.

## Claims

1. A process for preparing a toilet bar composition comprising the steps of:
(a) forming a mixture of cationic guar gum and alkali metal soap mix noodles wherein said guar gum is present at a level of from 0.2% to 5% by weight of said composition; said mixture containing from 8% to 12% by weight thereof of moisture;
(b) hydrating said cationic guar gum by milling said mixture into a uniform mixture;
(c) plodding said uniform mixture;
(d) extruding said milled and plodded mixture; and
(e) stamping said extruded mixture into said toilet bars,
characterised in that said cationic guar gum is a fast hydrating cationic guar gem which at a level of 2.6% in 9.5% alkaline (pH 9.4) salt (NaCl) water solution at 25°C has a viscosity of at least 800 mPa.s in 10 minutes and at least 1,000 mPa.s in 30 minutes under the same conditions.

2. The process of Claim 1 comprising from 0.5% to 4% by weight of the cationic guar gum.

3. The process of Claim 2 wherein said cationic guar gum is present at from 1% to 3% by weight of the bar.

4. The process of Claim 1 wherein said cationic guar gum is present from 1% to 2% by weight of the bar.

5. The process of Claim 1 wherein said alkali metal soap comprises a mixture of alkali metal tallow soap and alkali metal coconut soap.

6. The process of Claim 1 wherein said alkali metal soap comprises from 1 to 9 parts by weight alkali metal tallow soap per part by weight alkali metal coconut soap.

7. The process of Claim 1 wherein said cationic guar gum has a viscosity profile of:
from 5 to 500 mPa.s (cps) in 5 minutes;
from 1,000 to 5,000 mPa.s (cps) in 30 minutes;
from 3,000 to 9,000 mPa.s (cps)in 60 minutes; and
from 5,000 to 14,000 mPa.s (cps) in 120 minutes.

8. The process composition of Claim 1 wherein the toilet bar composition contains from 2% to 20% by weight of a synthetic surfactant selected from alkyl glyceryl ether sulfonates, anionic acyl sarcosinates, methyl acyl taurates, N-acyl glutamates, alkyl glucosides, acyl isethionates, alkyl sulfosuccinates, alkyl phosphate esters, ethoxylated alkyl phosphate esters, methyl glucose esters, protein condensates, mixtures of ethoxylated alkyl sulfates and alkyl amine oxides, betaines, sultaines, the alkyl ether sulfates with 1 to 12 ethoxy groups, and mixtures thereof, wherein said synthetic surfactants contain C₈-C₂₂ alkyl chains.

9. The process of Claim 1 wherein said composition contains up to 20% by weight of a C₁₀-C₁₈ alkyl glyceryl ether sulfonate.

## Patentansprüche

1. Verfahren zur Herstellung einer Toilettenriegelzusammensetzung, umfassend die Schritte von:
(a) Ausbilden eines Gemisches aus kationischem Guargummi und einer Alkalimetallseifenmischung in Nudelform, worin das genannte Guargummi in einer Menge von 0,2% bis 5%, bezogen auf das Gewicht der genannten Zusammensetzung vorliegt; welches Gemisch 8 Gew-.% bis 12 Gew.-% hievon an Feuchtigkeit enthält;
(b) Hydratisieren des genannten kationischen Guargummis durch Pillieren des genannten Gemisches Zu einem einheitlichen Gemisch;
(c) Strangpressen des genannten einheitlichen Gemisches;
(d) Extrudieren des genannten pillierten und stranggepreßten Gemisches; und
(e) Prägen des genannten extrudierten Gemisches zu Toilettenriegeln,
dadurch gekennzeichnet, daß das genannte kationische Guargummi ein schnell hydratisierendes kationisches Guargummi ist, welches in einer Menge von 2,6% in einer 9,5%igen wäßrigen Alkalisalzlösung (NaCl-Lösung) (pH-wert 9,4) bei 25°C nach 10 Minuten eine Viskosität von mindestens 800 mpa.s und unter den gleichen Bedingungen nach 30 Minuten von mindestens 1.000 mPa.s besitzt.

2. Verfahren nach Anspruch 1, umfassend 0,5 Gew.-% bis 4 Gew.-% des kationischen Guargummis.

3. Verfahren nach Anspruch 2, worin das genannte kationische Guargummi in einer Menge von 1 Gew.-% bis 3 Gew.-% des Riegels vorliegt.

4. Verfahren nach Anspruch 1, worin das genannte kationische Guargummi in einer Menge von 1 Gew.-% bis 2 Gew.-% des Riegels vorliegt.

5. Verfahren nach Anspruch 1, worin die genannte Alkalimetallseife ein Gemisch aus Alkalimetalltalgseife und Alkalimetallkokosnußseife umfaßt.

6. Verfahren nach Anspruch 1, worin die genannte Alkalimetallseife 1 bis 9 Gewichtsteile Alkalimetalltalgseife je Gewichtsteil an Alkalimetallkokosnußseife umfaßt.

7. Verfahren nach Anspruch 1, worin das genannte kationische Guargummi das folgende Viskositätsprofil besitzt:
von 5 auf 500 mPa.s (cPs) in 5 Minuten;
von 1.000 bis 5.000 mPa.s (cPs) in 30 Minuten;
von 3.000 bis 9.000 mPa.s (cPs) in 60 Minuten; und
von 5.000 bis 14.000 mPa.s (cPs) in 120 Minuten.

8. Zusammensetzung im Verfahren nach Anspruch 1, worin die Toilettenriegelzusammensetzung 2 Gew.-% bis 20 Gew.-% eines synthetischen grenzflächenaktiven Mittels enthält, welches unter Alkylglycerylethersulfonaten, anionischen Acylsarcosinaten, Methylacyltauraten, N-Acylglutamaten, Alkylglucosiden, Acylisethionaten, Alkylsulfosuccinaten, Alkylphosphatestern, ethoxylierten Alkylphosphatestern, Methylglucoseestern, Proteinkondensaten, den Gemischen aus ethoxylierten Alkylsulfaten und Alkylaminoxiden, Betainen, Sultainen, den Alkylethersulfaten mit 1 bis 12 Ethoxygruppen und Gemischen hievon ausgewählt ist, worin die genannten synthetischen grenzflächenaktiven Mittel C₈-C₂₂-Alkylketten aufweisen.

9. Verfahren nach Anspruch 1, worin die genannte Zusammensetzung bis zu 20 Gew.-% eines C₁₀-C₁₈-Alkylglycerylethersulfonates enthält.

## Revendications

1. Procédé de préparation d'une composition de savonnette, comprenant les étapes qui consistent à:
(a) former un mélange de gomme de guar cationique et de nouilles d'un mélange de savon de métal alcalin, dans lequel ladite gomme de guar est présente à un taux de 0,2% à 5% en poids de ladite composition; ledit mélange contenant de 8% à 12% en poids d'humidité;
(b) hydrater ladite gomme de guar cationique par broyage dudit mélange pour obtenir un mélange uniforme;
(c) boudiner ledit mélange uniforme;
(d) extruder ledit mélange broyé et boudiné; et
(e) estamper ledit mélange extrudé sous forme de savonnettes,
caractérisé en ce que ladite gomme de guar cationique est une gomme de guar cationique à hydratation rapide, qui possède, à un taux de 2,6% en solution aqueuse alcaline (pH 9,4) de sel (NaCl) à 9,5%, à 25°C, une viscosité d'au moins 800 mPa.s en 10 minutes et d'au moins 1000 mPa.s en 30 minutes dans les mêmes conditions.

2. Procédé selon la revendication 1, comprenant de 0,5% à 4% en poids de gomme de guar cationique.

3. Procédé selon la revendication 2, dans lequel ladite gomme de guar cationique est présente à raison de 1% à 3% en poids de la savonnette.

4. Procédé selon la revendication 1, dans lequel ladite gomme de guar cationique est présente à raison de 1% à 2% en poids de la savonnette.

5. Procédé selon la revendication 1, dans lequel ledit savon de métal alcalin comprend un mélange de savon de suif de métal alcalin et de savon de coprah de métal alcalin.

6. Procédé selon la revendication 1, dans lequel ledit savon de métal alcalin comprend de 1 à 9 parties en poids de savon de suif de métal alcalin par partie en poids de savon de coprah de métal alcalin.

7. Procédé selon la revendication 1, dans lequel ladite gomme de guar cationique possède le profil de viscosité suivant:
de 5 à 500 mPa.s (cP) en 5 minutes;
de 1000 à 5000 mPa.s (cP) en 30 minutes;
de 3000 à 9000 mPa.s (cP) en 60 minutes; et
de 5000 à 14.000 mPa.s (cP) en 120 minutes.

8. Procédé selon la revendication 1, dans lequel ladite composition de savonnette contient de 2% à 20% en poids d'un tensioactif synthétique choisi parmi les alkylglycéryl-éthersulfonates, les acyl-sarcosinates anioniques, les méthylacyltaurates, les N-acylglutamates, les alkylglucosides, les acyliséthionates, les alkylsulfosuccinates, les esters d'alkylphosphates, les esters d'alkylphosphates éthoxylés, les esters de méthylglucose, les produits de condensation de protéines, les mélanges d'alkylsulfates éthoxylés et d'oxydes d'alkylamines, les bétaïnes, les sultaïnes, les alkyléthersulfates possédant 1 à 12 groupes éthoxy, et leurs mélanges, dans laquelle lesdits tensioactifs synthétiques contiennent des chaînes alkyles en C₈-C₂₂.

9. Procédé selon la revendication 1 dans lequel ladite composition contient jusqu'à 20% en poids d'un alkyl (en C₁₀-C₁₈) glycéryl-éther-sulfonate.
